# FASCICULE DE BREVET EUROPEEN

(11) **EP 1 467 788 B1**
(45) Date de publication et mention de la délivrance du brevet: **15.09.2010**
(21) Numéro de dépôt: 02799112.4
(22) Date de dépôt: 23.12.2002
(51) Int. Cl.: A61M 15/00

(54) **DISPOSITIF DE DISTRIBUTION DE PRODUIT FLUIDE**
SPENDER FÜR FLÜSSIGE STOFFE
FLUID PRODUCT DISPENSING DEVICE

(30) Priorité: 28.12.2001 FR 0117027
(43) Date de publication de la demande: 20.10.2004
(73) Titulaire: Valois SAS, 27110 Le Neubourg (FR)
(72) Inventeur: STRADELLA, Giuseppe, I-16032 Camogli (GE) (IT)
(74) Mandataire: CAPRI
(86) Numéro de dépôt international: PCT/FR2002/004533
(87) Numéro de publication internationale: WO 2003/055548

(56) Documents cités:
- WO-A-01/70319
- WO-A-02/41939
- GB-A- 2 323 041
- US-A- 5 826 571
- US-B1- 6 328 035

## Description

La présente invention concerne un dispositif de distribution de produit fluide, et plus particulièrement un dispositif d'inhalation comportant une valve doseuse, du type MDI (« Metered Dose Inhaler »), dans lequel l'actionnement de la valve doseuse est commandé par l'inhalation de l'utilisateur.

De tels inhalateurs actionnés par l'inhalation, appelés généralement BAI (« Breath Actuated Inhaler »), utilisant des systèmes de valve doseuse (MDI), sont habituellement basés sur un mécanisme de déclenchement comportant un ressort, ledit ressort étant libéré par un dispositif approprié au moment de l'inhalation de l'utilisateur. Le chargement ou compression du ressort est réalisé habituellement en actionnant un levier, par exemple lors de l'ouverture du couvercle de l'embout buccal de l'inhalateur, et la force du ressort est dirigée, au moment de l'inhalation, contre la valve doseuse de l'inhalateur ou contre le réservoir, permettant l'actionnement de la valve en déplaçant la soupape de la valve par rapport au réservoir. Ceci est rendu possible du fait que l'élément parmi la soupape de la valve et le réservoir qui n'est pas soumis à l'action du ressort préalablement armé, est maintenu fixe à l'intérieur du dispositif. Après son actionnement et suite à la distribution du produit fluide contenu dans le réservoir, la valve doseuse reste généralement comprimée, avec la soupape dans sa position d'actionnement, jusqu'à ce que la charge du ressort est relâchée, ce qui ne se produit que lorsque le couvercle de l'embout buccal est refermé.

La structure décrite ci-dessus, est la source d'un problème qui est lié à la manière dans laquelle la plupart des valves doseuse fonctionnent. Ces valves comprennent généralement un ressort de rappel et une chambre de dosage qui est remplie par le mélange constitué du produit fluide, en général un médicament, et du gaz propulseur liquéfié. Le remplissage de la chambre de dosage est réalisé par gravité et seulement lorsque la soupape de la valve se déplace de sa position de distribution vers sa position de repos, c'est à dire lorsque la force appliquée sur la valve par le ressort du système de déclenchement est relâchée. Ceci implique donc que la tension du ressort doit être libéré lorsque le dispositif est dans une position appropriée pour permettre un remplissage par gravité de la chambre de dosage de la valve. La position requise pour un remplissage efficace et complet de la chambre de dosage est la position d'utilisation de l'inhalateur, dans laquelle le réservoir est généralement disposé au-dessus de la valve doseuse, l'utilisateur ayant l'embout buccal dans sa bouche pour inspirer la dose de produit distribué.

Dès la fin de la distribution de la dose de produit, lorsque l'utilisateur ressort le dispositif de sa bouche, la probabilité est grande que l'inhalateur ne soit plus dans la position requise pour un remplissage effectif, et le risque est grand que l'utilisateur referme le couvercle de l'embout buccal alors que le dispositif d'inhalation se trouve dans une position non appropriée pour un remplissage total de la chambre de dosage.

Il a donc été proposé des systèmes pour libérer la soupape et permettre son retour automatique vers sa position de repos, indépendamment de toute intervention de l'utilisateur. Ces systèmes comportent généralement des moyens actionnés lorsque la soupape atteint sa position de distribution, et qui permettent un retour immédiat de la soupape vers sa position de repos et donc un remplissage de la chambre de dosage dans la position adéquate. Le document US-5 826 571 divulgue un système tel que décrit dans le préambule de la revendication 1.

L'utilisation de tels systèmes de libération de soupape peut toutefois entraîner d'autres inconvénients.

En effet, il est nécessaire d'allouer un temps suffisant pour permettre l'expulsion de la totalité de la dose hors de la chambre de dosage, lors de l'actionnement. Or, généralement, la distribution du produit a lieu lorsque l'orifice d'entrée de la soupape est à l'intérieur de la chambre de dosage. Ceci se produit en fin de course d'actionnement de la soupape et au début de sa course de retour vers sa position de repos. Ainsi, les systèmes de libération de soupape, qui ramènent celle-ci vers sa position de repos dès qu'elle a atteint sa position de distribution, risquent de diminuer le temps de vidage de la chambre de dosage de manière trop importante. En d'autres mots, la soupape risque d'être libérée et ramenée vers sa position de repos avant que la totalité de la dose ait été distribuée. La précision du dosage peut en être affectée, ce qui peut avoir des conséquences très graves, notamment avec certains produits pharmaceutiques dont l'efficacité est directement liée à la précision des doses distribuées. Le document WO 01/70319 divulgue un dispositif comportant des moyens pour bloquer temporairement la soupape en position de distribution. Ce système présente l'inconvénient de maintenir la valve ouverte pendant ce blocage temporaire, ce qui présente un risque si l'utilisateur bouge le dispositif avant fermeture de la valve. Le document WO 02/41 939, opposable seulement à la nouveauté, décrit un système permettant de ramener automatiquement la soupape vers sa position de repos.

La présente invention a pour but de fournir un dispositif de distribution de produit fluide qui ne reproduit pas les inconvénients susmentionnés.

La présente invention a donc pour but de fournir un dispositif de distribution de produit fluide qui garantit un vidage total de la chambre de dosage de la valve à chaque actionnement.

La présente invention a aussi pour but de fournir un dispositif de distribution de produit fluide qui garantit un remplissage total de la chambre de dosage de la valve après chaque actionnement.

La présente invention a également pour but de fournir un tel dispositif de distribution de produit fluide qui permet un remplissage total et un vidage total de la chambre de dosage de la valve doseuse, indépendamment de l'utilisateur.

La présente invention a aussi pour but de fournir un tel dispositif de distribution de produit fluide qui soit simple et peu coûteux à fabriquer et à assembler.

La présente invention a donc pour objet un dispositif de distribution de produit fluide tel que décrit dans la revendication 1. Des modes de réalisation avantageuse sont décrits dans les revendications dépendantes.

D'autres caractéristiques et avantages de la présente invention apparaîtront plus clairement au cours de la description détaillée suivante de deux modes de réalisation de celle-ci, décrits en référence aux dessins joints, et donnés à titres d'exemples non limitatifs, sur lesquels :
- la figure 1 est une vue schématique en section transversale d'un dispositif de distribution de produit fluide selon un premier mode de réalisation de la présente invention, avant actionnement de la valve,
- la figure 2 est une vue similaire à celle de la figure 1, après actionnement de la valve,
- la figure 3 est une vue schématique en section transversale partiellement découpée d'un second mode de réalisation de l'invention, avant actionnement de la valve, et
- la figure 4 est une vue similaire à celle de la figure 3, après actionnement de la valve.

La présente invention s'applique à tous types d'inhalateurs déclenchés par l'inhalation de l'utilisateur (BAI), et même si la description de plusieurs modes de réalisation va être faite en connexion avec un inhalateur dans lequel le système de déclenchement par l'inhalation agit sur la soupape de la valve, il est clair qu'elle s'applique également aux dispositifs dans lesquels le ressort agit sur le réservoir. Que la force élastique d'actionnement de la valve soit exercée sur la soupape ou sur le réservoir n'a pas d'influence directe sur la présente invention qui s'applique dans les deux cas, l'objet de la présente invention étant de ralentir le déplacement de la soupape vers sa position de distribution.

La description ci-après va donc être faite en référence à un dispositif du type divulgué dans le document WO 99/44662 qui concerne le fonctionnement du système de déclenchement par l'inhalation du dispositif de distribution de produit fluide.

Selon l'invention, on prévoit un dispositif de frein agissant sur l'élément parmi la soupape de la valve et le réservoir qui est soumis à la force élastique de l'élément d'actionnement 10 du système de déclenchement, c'est à dire sur l'élément qui se déplace lors de l'actionnement. Ce dispositif de frein permet donc un déplacement ralenti de manière prédéterminée de la partie du BAI mobile pendant l'actionnement de la valve.

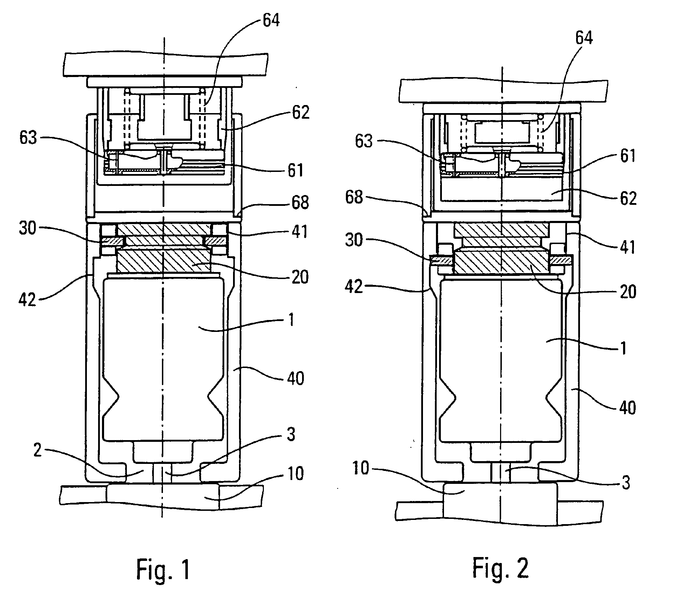

En référence aux figures 1 et 2, il est représenté un premier mode de réalisation, dans lequel le frein est un frein pneumatique 60. Ce frein 60 comporte un piston 61 relié à un élément de commande 40 qui coopère avec l'élément d'actionnement 10. L'élément de commande 40 peut soit être directement relié audit piston 61, soit on peut prévoir un élément intermédiaire 68 coopérant d'une part avec l'élément de commande 40 et d'autre part avec le piston 61, comme représenté sur les figures 1 et 2. Ledit piston 61, qui comporte de préférence un revêtement en élastomère, coulisse de manière étanche dans une chambre 62, ladite chambre 62 ou ledit piston 61 étant pourvus d'un petit passage ou orifice 63 de faible diamètre.

Dans l'exemple représenté sur les figures 1 et 2, le frein 60 fonctionne par dépression, c'est à dire que dans la position de repos représentée sur la figure 1, le piston 61 est disposé contre la paroi d'extrémité formant la chambre 62. Lorsque l'utilisateur actionne le dispositif, l'élément d'actionnement 10 du système de déclenchement agit sur l'élément de commande 40 qui entraîne le piston à se déplacer en éloignement de la paroi d'extrémité de la chambre 62, créant une dépression entre cette paroi d'extrémité et ledit piston 61, l'orifice 63 de petit diamètre ne permettant une pénétration d'air à l'intérieur de ladite chambre 62 qu'à faible vitesse. Ainsi, ledit piston 61, et donc la soupape 3, ne peuvent se déplacer que lentement, assurant ainsi le freinage requis. Bien entendu, l'efficacité du frein dépend des dimensions de l'orifice 63 et de la chambre 62.

Avantageusement, le frein pneumatique est également pourvu d'un ressort de rappel 64 qui permet un retour du piston dans sa position de départ lorsque l'élément d'actionnement 10 du système de déclenchement est ramené dans sa position de repos (notamment par fermeture du couvercle).

Bien que l'exemple du frein pneumatique a été décrit en référence à un système fonctionnant à dépression, il est évident que le frein pneumatique des figures 1 et 2 peut être réalisé de manière à fonctionner par compression. Dans ce cas, le piston 61 serait, dans la position de repos, en éloignement de la paroi d'extrémité de la chambre 62 et lorsque le dispositif est actionné, il serait sollicité élastiquement en direction de cette paroi d'extrémité de sorte que l'air contenu à l'intérieur de la chambre 62 ne pourrait s'échapper qu'à travers ledit orifice de petit diamètre 63, ce qui ne peut se faire que lentement, fournissant ainsi le freinage requis.

D'autre part, on peut également envisager de réaliser le système de frein de manière hydraulique, en remplaçant l'air par un liquide quelconque souhaité, et en adaptant de manière correspondante les dimensions de la chambre 62 et de l'orifice de petit diamètre 63.

En variante, on pourrait aussi utiliser un système d'engrenage permettant de fournir la fonction de freinage souhaitée.

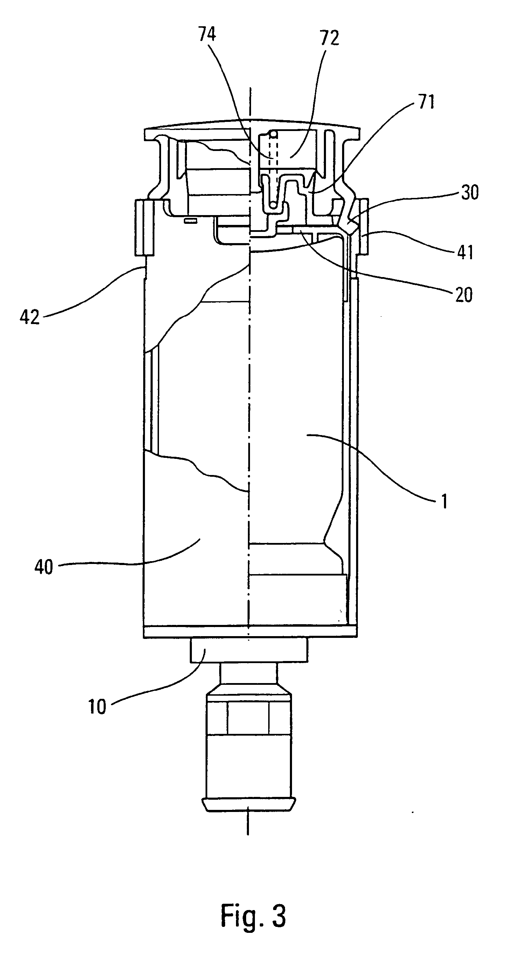

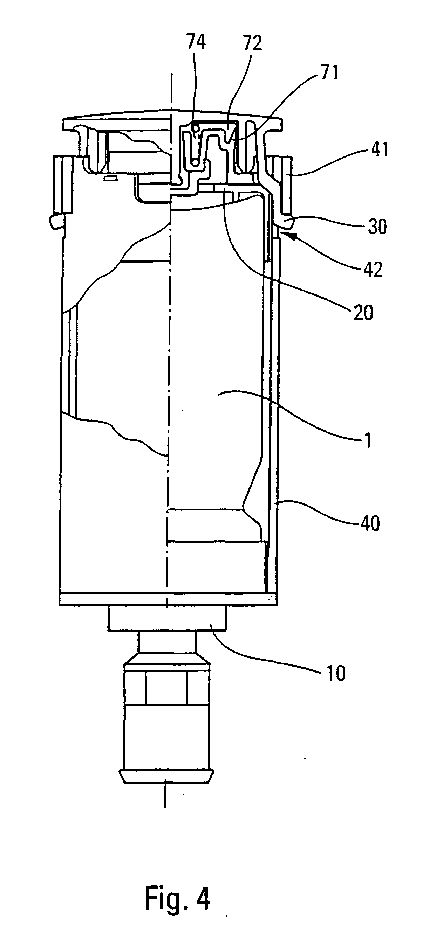

Les figures 3 et 4 montrent un second mode de réalisation du dispositif de frein, dans lequel le frein 70 comporte un piston 71 coulissant de manière non-étanche dans la chambre 72. De préférence, ce piston 71 ne comporte pas de joint en élastomère et un passage de fuite se forme entre le piston 71 et la chambre 72, l'air contenu dans la chambre 72 pouvant ainsi s'écouler lentement hors de celle-ci à travers ce passage de fuite. Avantageusement, le piston 71 comporte aussi un ressort de rappel 74. L'exemple des figures 3 et 4 est donc un frein à compression. Ceci est avantageux, car l'efficacité du frein est alors maximale en fin de course d'actionnement de la soupape 3, c'est à dire au moment où la soupape entre en communication avec la chambre de dosage de la valve. La fin de la course d'actionnement de la soupape 3 est donc freinée de manière très efficace, ce qui rallonge d'autant le temps pendant lequel la chambre de dosage peut se vider. On assure ainsi un vidage total de la chambre de dosage à chaque actionnement et donc une précision optimale du dosage, même avec l'utilisation d'un système de libération de soupape, comme cela sera décrit ci-après.

Selon l'invention, on combine en effet avantageusement le système de frein décrit en référence aux figures 1 à 4 ci-dessus, avec un système de libération de soupape agissant automatiquement à partir du moment où la soupape 3 de la valve arrive dans sa position de distribution. Cette combinaison permet d'assurer que la soupape revient automatiquement dans sa position de repos immédiatement après chaque actionnement, pour garantir un remplissage totale et fiable de la chambre de dosage de la valve.

En référence aux figures 1 et 2, il va être décrit une première variante de réalisation d'un tel système de libération de soupape.

La figure 1 représente très schématiquement un réservoir de produit fluide 1 sur lequel est monté une valve doseuse 2 d'une manière quelconque souhaitée, ladite valve doseuse 2 comprenant une soupape 3 mobile entre une position de repos et une position de distribution. La valve doseuse 2 comporte une chambre de dosage (non représentée) qui est vidée lorsque la soupape 3 est dans sa position de distribution et qui se remplit par gravité lorsque la soupape 3 revient de sa position de distribution vers sa position de repos. Ladite soupape 3 coopère avec un élément d'actionnement 10 qui fait de préférence partie d'un système de déclenchement par l'inhalation et qui est constitué par ou est solidaire d'un ressort (non représenté sur les figures 1 et 2), ledit ressort pouvant être armé par l'utilisateur avant l'utilisation du dispositif de sorte que lors de l'inhalation, l'élément d'actionnement 10 est libéré et peut exercer une force sur la soupape 3 pour actionner la valve doseuse. Pendant ce processus pendant lequel la soupape 3 se déplace de sa position de repos vers sa position d'actionnement, le réservoir 1 est maintenu fixe dans le corps du dispositif.

Le système de libération de soupape comporte un élément de blocage 20, qui dans l'exemple des figures 1 et 2 coopère avec le fond du réservoir 1. Cet élément de blocage 20 est retenu dans sa position de blocage par un organe de retenue 30, qui dans l'exemple représenté sur les figures 1 et 2 est réalisé sous la forme d'un anneau fendu pouvant se déformer radialement vers l'extérieur. Cet anneau fendu 30 coopère avec ledit élément de blocage 20 pour le maintenir dans sa position de blocage, dans laquelle il maintient le réservoir 1 fixe à l'intérieur du dispositif. Un élément de commande 40, qui est avantageusement le même que celui du frein décrit précédemment, est relié d'une part à l'élément d'actionnement 10 du système de déclenchement, et coopère d'autre part avec ledit élément de retenue 30. Ainsi, comme visible sur les figures 1 et 2, l'élément de commande 40 est déplacé en même temps que l'élément d'actionnement 10, et donc en même temps que la soupape 3 lorsque la valve doseuse est actionnée. Cet élément de commande 40 peut être réalisé sous la forme d'un manchon qui entoure de manière externe ledit organe de retenue 30 et comporte un premier diamètre 41 et un second diamètre 42 supérieur audit premier diamètre. Le premier diamètre 41 de l'élément de commande 40 coopère avec l'organe de retenue 30 avant l'actionnement du dispositif, et le second diamètre 42 coopère avec ledit organe de retenue 30 après l'actionnement du dispositif, lorsque la soupape 3 est dans sa position de distribution. A ce moment là, l'organe de retenue 30 peut se déformer radialement vers l'extérieur à l'intérieur du second diamètre 42 de l'élément de commande 40 pour libérer l'élément de blocage 20. L'élément de blocage 20 peut alors coulisser axialement sous l'effet de la force exercée par le ressort de rappel (non représenté) de la valve doseuse, de sorte que celle-ci revient vers sa position de repos dès que l'organe de retenue 30 est déplacé vers sa position de non retenue représentée sur la figure 2. La soupape 3 est toujours bloquée par l'élément d'actionnement 10 du système de déclenchement, tant que celui-ci n'est pas ramené vers sa position de repos, mais c'est le réservoir 1 qui peut alors librement se déplacer pour permettre à la soupape 3 de revenir vers sa position de repos après distribution du produit. C'est donc l'élément qui est fixe lors de l'actionnement, en l'occurrence le réservoir 1 dans cet exemple, qui se déplace pour libérer la soupape 3.

Ainsi, dans l'exemple de réalisation représenté sur les figures 1 et 2, au moment où la soupape 3 atteint sa position de distribution et délivre le produit contenu dans la chambre de dosage de la valve doseuse 2, le réservoir 1 est libéré, l'élément de blocage 20 pouvant se déplacer vers sa position de déblocage, la soupape 3 revenant alors vers sa position de repos, ce qui permet un remplissage de la chambre de dosage alors que le dispositif est toujours dans la bouche de l'utilisateur, et garantissant que ce remplissage se produit dans la position requise, comme représentée sur les dessins, avec la valve doseuse 2 disposée en dessous du réservoir 1, le remplissage étant réalisé par gravité.

De manière avantageuse, le remplissage est réalisé dès la fin de la distribution de la dose précédente, c'est à dire très rapidement. Ceci permet d'éviter tout problème de surdosage qui pourrait se produire en cas d'attente en position inversée, notamment avec des suspensions.

Avantageusement, on peut prévoir un ressort de rappel pour l'élément de blocage 20 et un ressort de rappel pour l'élément de commande 40, de sorte que lorsque l'utilisateur ramène l'élément d'actionnement 10 vers sa position de repos, l'élément de commande 40 est ramené automatiquement vers sa position initiale par ledit ressort de rappel 44, de même que l'élément de blocage 20 est ramené vers sa position de blocage par le ressort de rappel 24, l'organe de retenue 30 revenant se positionner à l'intérieur de la rainure dudit élément de blocage 20 pour bloquer ledit élément de blocage en position de blocage, et le premier diamètre 41 de l'organe de commande venant bloquer l'organe de retenue en position de retenue.

En référence aux figures 3 et 4, il est représenté une variante de réalisation du système de libération représenté sur les figures 1 et 2. Dans cette variante, l'organe de retenue est formé par une ou plusieurs pattes élastiques 30 déformables élastiquement, de préférence vers l'extérieur. La partie de second diamètre 42 de l'élément de commande 40 est formée par une ou plusieurs ouvertures correspondantes permettant auxdites pattes de s'écarter pour ainsi libérer l'élément de blocage 20.

Bien que la présente invention ait été décrite en référence à plusieurs modes de réalisation de celle-ci, qui sont donnés à titres d'exemples non limitatifs, il est clair que l'homme du métier peut y apporter plusieurs modifications sans sortir du cadre de la présente invention défini par les revendications annexées.

## Revendications

1. Dispositif de distribution de produit fluide comportant un réservoir (1) contenant du produit fluide et un gaz propulseur, une valve doseuse (2), montée sur ledit réservoir, comportant une chambre de dosage et une soupape (3) mobile entre une position de repos et une position de distribution, et un système de déclenchement automatique, de préférence actionné par l'inhalation de l'utilisateur, pour actionner ladite valve, ledit système de déclenchement comportant un élément d'actionnement (10), adapté à déplacer l'un parmi la soupape (3) et le réservoir (1) par rapport à l'autre, pour amener la soupape (3) de la valve (2) dans sa position de distribution, le dispositif comportant en combinaison un système de frein (60, 70) qui coopère avec le réservoir (1) ou la soupape (3) de la valve (2) pour ralentir le déplacement de la soupape (3) vers sa position de distribution, lors de l'actionnement du dispositif, et un système de libération de soupape, actionné automatiquement lorsque la soupape (3) atteint sa position de distribution, et qui ramène ladite soupape (3) dans sa position de repos indépendamment de la position dudit élément d'actionnement (10), **caracterisé en ce que** le système de frein est configuré pour agir tout le long de l'actionnement du dispositif.

2. Dispositif selon la revendication 1, dans lequel ledit dispositif de frein (60, 70) est pneumatique et/ou hydraulique.

3. Dispositif selon la revendication 2, dans lequel ledit dispositif de frein (60) comporte un piston (61) relié audit élément d'actionnement (10) par l'intermédiaire d'un élément de commande (40), ledit piston (61) coulissant de manière étanche dans une chambre (62), ladite chambre (62) ou ledit piston (61) étant pourvu(e) d'un petit passage (63), de sorte que du gaz ou du liquide ne peut s'écouler que lentement dans ou hors de ladite chambre (62), assurant un déplacement lent dudit piston (61).

4. Dispositif selon la revendication 2, dans lequel ledit dispositif de frein (70) comporte un piston (71) relié audit élément d'actionnement (10) par l'intermédiaire d'un élément de commande (40), ledit piston (71) coulissant de manière non-étanche dans une chambre (72) de telle sorte que l'air contenu dans la chambre (72) ne peut s'écouler que lentement hors de ladite chambre (72), assurant un déplacement lent dudit piston (71).

5. Dispositif selon l'une quelconque des revendications précédentes, dans lequel ledit système de libération de soupape comporte un élément de blocage (20) coopérant avec l'un parmi la soupape (3) de la valve et le réservoir (1) de produit, ledit élément de blocage (20) étant mobile entre une position de blocage, dans laquelle la soupape (3) peut être amenée dans sa position de distribution par ledit élément d'actionnement (10) du système de déclenchement, et une position de déblocage, dans laquelle la soupape (3) est ramenée dans sa position de repos indépendamment de la position dudit élément d'actionnement (10), ledit élément de blocage (20) étant sollicité vers sa position de déblocage après actionnement du système de déclenchement, lorsque la soupape (3) atteint sa position de distribution.

6. Dispositif selon la revendication 5, dans lequel le système de libération de soupape comporte un organe de retenue (30) déplaçable entre une position de retenue dans laquelle il retient ledit élément de blocage (20) dans sa position de blocage et une position de non-retenue, dans laquelle il ne retient pas ledit élément de blocage (20) dans sa position de blocage, ledit organe de retenue (30) étant déplacé vers sa position de non-retenue lorsque la soupape (3) atteint sa position de distribution.

7. Dispositif selon la revendication 6, dans lequel ledit système de libération de soupape comporte un élément de commande (40) coopérant d'une part avec la soupape (3) de la valve et/ou l'élément d'actionnement (10), et d'autre part avec ledit organe de retenue (30), de telle sorte que lorsque la soupape (3) arrive dans sa position de distribution, l'élément de commande (40) permet le déplacement de l'organe de retenue (30) dans sa position de non-retenue, de sorte que l'élément de blocage (20) est déplacé vers sa position de déblocage et la soupape est ramenée dans sa position de repos par le ressort de rappel de la valve.

8. Dispositif selon la revendication 7, dans lequel ledit organe de retenue (30) est élastiquement déformable et ledit élément de commande comporte un premier diamètre interne (41) coopérant avec l'organe de retenue (30) pour empêcher sa déformation et ainsi le maintenir dans sa position de retenue, et un second diamètre interne (42) supérieur audit premier diamètre interne (41), qui coopère avec ledit organe de retenue (30) lorsque la soupape (3) atteint sa position de distribution, permettant alors la déformation dudit organe de retenue (30) vers sa position de non retenue.

9. Dispositif selon la revendication 8, dans lequel ledit organe de retenue (30) comporte une ou plusieurs pattes (30) élastiquement déformables.

10. Dispositif selon la revendication 8 ou 9, dans lequel la partie de second diamètre (42) de l'élément de commande (40) est formée par une ou plusieurs ouverture(s) adaptée(s) à coopérer avec l'organe de retenue (30).

## Claims

1. A fluid dispenser device comprising: a reservoir (1) containing the fluid and a propellant; a metering valve (2) mounted on said reservoir and comprising a metering chamber, and a valve member (3) that is movable between a rest position and a dispensing position; and an automatic trigger system, preferably actuated by the user inhaling in order to actuate said valve, said trigger system including an actuator element (10) adapted to displace one of the valve member (3) and the reservoir (1) relative to the other so as to bring the valve member (3) of the valve (2) into its dispensing position, the device including, in combination, a brake system (60, 70) which co-operates with the reservoir (1) or with the valve member (3) in order to slow down displacement of the valve member (3) towards its dispensing position during actuation of the device, and a valve-member release system that is actuated automatically when the valve member (3) reaches its dispensing position, and which returns said valve member (3) to its rest position independently of the position of said actuator element (10), **characterized in that** the brake system is acting all along the actuation of the device.

2. A device according to claim 1, in which said brake device (60, 70) is pneumatic and/or hydraulic.

3. A device according to claim 2, in which said brake device (60) comprises a piston (61) connected to said actuator element (10) by means of a control element (40), said piston (61) sliding in sealed manner in a chamber (62), said chamber (62) or said piston (61) being provided with a small passage (63) so that the gas or liquid can flow only slowly into or out of said chamber (62), ensuring that said piston (61) is displaced slowly.

4. A device according to claim 2, in which said brake device (70) comprises a piston (71) connected to said actuator element (10) by means of a control element (40), said piston (71) sliding in non-sealed manner in a chamber (72) so that the air contained in the chamber (72) can flow only slowly out of said chamber (72), ensuring that said piston (71) is displaced slowly.

5. A device according to any preceding claim, in which said valve-member release system includes a blocking element (20) co-operating with one of the valve member (3) and the fluid reservoir (1), said blocking element (20) being movable between a blocking position, in which the valve member (3) can be brought into its dispensing position by said actuator element (10) of the trigger system, and an unblocking position, in which the valve member (3) is returned to its rest position independently of the position of said actuator element (10), said blocking element (20) being urged towards its unblocking position after the trigger system has been actuated, when the valve member (3) reaches its dispensing position.

6. A device according to claim 5, in which the valve-member release system includes a retaining member (30) that is displaceable between a retaining position, in which it retains said blocking element (20) in its blocking position, and a non-retaining position, in which it does not retain said blocking element (20) in its blocking position, said retaining member (30) being displaced towards its non-retaining position when the valve-member (3) reaches it dispensing position.

7. A device according to claim 6, in which said valve-member release system includes a control element (40) co-operating firstly with the valve member (3) and/or with the actuator element (10), and secondly with said retaining member (30), so that when the valve member (3) reaches its dispensing position, the control member (40) makes it possible to displace the retaining member (30) into its non-retaining position, so that the blocking element (20) is displaced towards it unblocking position and the valve member is returned to its rest position by the return spring of the valve.

8. A device according to claim 7, in which said retaining member (30) is elastically deformable, and said control element includes a first inside diameter (41) co-operating with the retaining member (30) so as to prevent it from deforming, and thus hold it in its retaining position, and a second inside diameter (42) that is greater than said first inside diameter (41), which co-operates with said retaining member (30) when the valve member (3) reaches its dispensing position, thereby enabling said retaining member (30) to deform towards its non-retaining position.

9. A device according to claim 8, in which said retaining member (30) includes one or a plurality of elastically deformable tabs (30).

10. A device according to claim 8 or claim 9, in which the second-diameter portion (42) of the control element (40) is formed by one or a plurality of openings adapted to co-operate with the retaining member (30).

## Patentansprüche

1. Ausgabevorrichtung für ein fluides Produkt, aufweisend einen Behälter (1), der fluides Produkt und ein Treibgas enthält, ein Dosierventil (2), das auf dem Behälter montiert ist, aufweisend eine Dosierkammer und ein Ventilelement (3), das zwischen einer Ruheposition und einer Ausgabeposition beweglich ist, und ein automatisches Auslösesystem, das vorzugsweise durch Inhalation des Benutzers betätigt wird, um das Ventil zu betätigen, wobei das Auslösesystem ein Betätigungselement (10) aufweist, das geeignet ist, das Ventilelement (3) und den Behälter (1) zueinander zu verschieben, um das Ventilelement (3) des Ventils (2) in seine Ausgabeposition zu überführen, wobei die Vorrichtung in Kombination ein Bremssystem (60, 70), das mit dem Behälter (1) oder dem Ventilelement(3) des Ventils (2) zusammenwirkt, um bei der Betätigung der Vorrichtung die Verschiebung des Ventilelements (3) in seine Ausgabeposition zu verlangsamen, und ein Ventilelementfreigabesystem aufweist, das automatisch betätigt wird, wenn das Ventilelement (3) seine Ausgabeposition erreicht, und das das Ventilelement (3) unabhängig von der Position des Betätigungselements (10) in seine Ruheposition überführt, **dadurch gekennzeichnet, dass** das Bremssystem dafür konfiguriert ist, entlang der gesamten Betätigung der Vorrichtung wirksam zu sein.

2. Vorrichtung nach Anspruch 1, wobei die Bremsvorrichtung (60, 70) pneumatisch und/oder hydraulisch ist.

3. Vorrichtung nach Anspruch 2, wobei die Bremsvorrichtung (60) einen Kolben (61) aufweist, der über ein Steuerelement (40) mit dem Betätigungselement (10) verbunden ist, wobei der Kolben (61) dichtend in einer Kammer (62) gleitet, wobei die Kammer (62) oder der Kolben (61) mit einem kleinen Durchgang (63) versehen ist, so dass Gas oder Flüssigkeit nur langsam in die oder aus der Kammer (62) fließen kann, wodurch ein langsames Verschieben des Kolbens (61) gewährleistet ist.

4. Vorrichtung nach Anspruch 2, wobei die Bremsvorrichtung (70) einen Kolben (71) aufweist, der über ein Steuerelement (40) mit dem Betätigungselement (10) verbunden ist, wobei der Kolben (71) nicht dichtend derart in einer Kammer (72) gleitet, dass die in der Kammer (72) enthaltene Luft nur langsam aus der Kammer (72) fließen kann, wodurch eine langsame Verschiebung des Kolbens (71) gewährleistet ist.

5. Vorrichtung nach einem der vorhergehenden Ansprüche, wobei das Ventilelementfreigabesystem ein Sperrelement (20) aufweist, das mit dem Ventilelement (3) des Ventils und dem Produktbehälter (1) zusammenwirkt, wobei das Sperrelement (20) zwischen einer Sperrposition, in der das Ventilelement (3) durch das Betätigungselement (10) des Auslösesystems in seine Ausgabeposition überführt werden kann, und einer Entsperrposition, in der das Ventilelement (3) unabhängig von der Position des Betätigungselements (10) in seine Ruheposition überführt wird, beweglich ist, wobei das Sperrelement (20) nach Betätigung des Auslösesystems in seine Entsperrposition beansprucht wird, wenn das Ventilelement (3) seine Ausgabeposition erreicht.

6. Vorrichtung nach Anspruch 5, wobei das Ventilelementfreigabesystem eine Rückhalteeinrichtung (30) aufweist, die zwischen einer Rückhalteposition, in der sie das Sperrelement (20) in seiner Sperrposition hält, und einer nicht rückhaltenden Position, in der sie das Sperrelement (20) nicht in seiner Sperrposition hält, verschiebbar ist, wobei die Rückhalteeinrichtung (30) in ihre nicht rückhaltende Position verschoben wird, wenn das Ventilelement (3) seine Ausgabeposition erreicht.

7. Vorrichtung nach Anspruch 6, wobei das Ventilelementfreigabesystem ein Steuerelement (40) aufweist, das einerseits mit dem Ventilelement (3) des Ventils und/oder dem Betätigungselement (10), und andererseits mit der Rückhalteeinrichtung (30) zusammenwirkt, so dass, wenn das Ventilelement (3) seine Ausgabeposition erreicht, das Steuerelement (40) die Verschiebung der Rückhalteeinrichtung (30) in ihre nicht rückhaltende Position ermöglicht, so dass das Sperrelement (20) in seine Entsperrposition verschoben wird und das Ventilelement durch die Rückstellfeder des Ventils in seine Ruheposition überführt wird.

8. Vorrichtung nach Anspruch 7, wobei die Rückhalteeinrichtung (30) elastisch verformbar ist und das Steuerelement einen ersten Innendurchmesser (41), der mit der Rückhalteeinrichtung (30) zusammenwirkt, um deren Verformung zu verhindern und sie so in ihrer Rückhalteposition zu halten, und einen zweiten Innendurchmesser (42) aufweist, der größer ist als der erste Innendurchmesser (41) und der mit der Rückhalteeinrichtung (30) zusammenwirkt, wenn das Ventilelement (3) seine Ausgabeposition erreicht, wodurch dann die Verformung der Rückhalteeinrichtung (30) in ihre nicht rückhaltende Position ermöglicht wird.

9. Vorrichtung nach Anspruch 8, wobei die Rückhalteeinrichtung (30) eine oder mehrere Klammern (30) aufweist, die elastisch verformbar sind.

10. Vorrichtung nach Anspruch 8 oder 9, wobei der Teil des zweiten Durchmessers (42) des Steuerelements (40) durch eine oder mehrere angepasste Öffnung(en) gebildet ist, die dazu geeignet sind, mit der Rückhalteeinrichtung (30) zusammenzuwirken.
